# EUROPEAN PATENT APPLICATION

(11) **EP 1 088 888 A1**
(43) Date of publication of application: **04.04.2001**
(21) Application number: 99919563.9
(22) Date of filing: 13.05.1999
(51) Int. Cl.: C12N 15/09, C12N 15/62, C12N 5/10, C12P 21/02, C07K 19/00

(54) **FUSED PROTEIN**

(30) Priority: 14.05.1998 JP 13238998
(71) Applicant: MERCK PATENT GmbH, 64293 Darmstadt (DE)
(72) Inventor: SUDO, Yukio, Fuji Photo Film Co., Ltd., Asaka-shi, Saitama 351-0024 (JP); YAMAMOTO, Masayoshi, Fuji Photo Film Co., Ltd., Minami-ashigara-shi, Kanagawa 250-0193 (JP); ORIKASA, Atsushi, Fuji Photo Film Co., Ltd., Asaka-shi, Saitama 351-0024 (JP); INOMATA, Hiroko, Fuji Photo Film Co., Ltd., Asaka-shi, Saitama 351-0024 (JP); TAKESHITA, Yumiko, Fuji Photo Film Co., Ltd., Asaka-shi, Saitama 351-0024 (JP)
(86) International application number: JP9902480
(87) International publication number: WO9958662

(57) **Abstract**

A fusion protein containing a target protein which comprises, from its N-terminal to C-terminal direction: a) a signal sequence; b) an immunoglobulin Fc region which lacks at least the CH1 domain; c) a peptide linker which comprises an enzymatic cleavage site that is cleavable with enterokinase and the like at its C-terminal; and d) an amino acid sequence of the target protein such as erythropoietin, wherein, after an enzymatic cleavage, the target protein does not contain at its N-terminal one or more amino acid residues derived from the peptide linker. A target protein having no amino acid modification at its N-terminal can be efficiently produced from the fusion protein by an enzymatic treatment.

## Description

### Technical Field

The present invention relates to a fusion protein that enables efficient production of a target protein, and a DNA encoding the fusion protein.

### Background Art

Recombinant protein expression systems utilizing the fusion protein technique have been conventionally used to ensure efficient protein production in Escherichia coli systems. A bacterial protein that is expressed at a very high level is generally used as a partner of a fusion protein, which achieves efficient transcription of the message, and in some cases enhances secretion and solubilization of the fusion protein (Biotechnology, 6, 1204, 1988; Biotechnology, 11, 187, 1993; Methods in Enzymology, 185, 129-161, 1990).

The major goal of the expression of recombinant fusion proteins in mammals has been to confer novel properties to the fusion molecules, e.g., protein A binding property, increased blood half-life, complement fixation property and the like, and accordingly, it has scarcely been studied to date from a viewpoint of efficient production (Trends in Biotechnology, 14, 52, 1996).

It has been reported that efficient expression and secretion of a target protein can be achieved by expression of a target protein fused with C-terminal of an immunoglobulin Fc region (Gillies et al., the specification of U.S. Patent No. 5,541,087). One of typical cassette vectors used in the method has a DNA sequence encoding, from its 5' to 3' direction, an immunoglobulin L chain signal sequence and an immunoglobulin Fc chain, whose 3' side can be inserted with a DNA having Xma restriction site (a DNA encoding a target protein). Another cassette vector is bound at its 3' side to a peptide linker that has a cleavage side of a proteolytic enzyme or the like. After the expression of the fusion protein by using the cassette vector, the target protein can be recovered by the cleavage of the fusion protein with a proteolytic enzyme such as enterokinase.

In Examples of the specification of the aforementioned United States patent, a cassette vector is disclosed, as a specific example according to the second embodiment of the cassette vector, in which an enzymatic cleavage site of enterokinase (DDDDK: amino acids are represented by one-letter symbols) was introduced at the 5' side of DNA encoding a target protein. The vector utilized the DNA set out below as a DNA encoding a portion of a peptide linker to provide for the enzymatic cleavage site into the expression cassette. For the ligation of the DNA with a DNA encoding a target protein, it was necessary to incorporate the Hind III cleavage site (AAGCTT) at its 5' end.

CCG GGT TCA GGG GAT GAC GAT GAC GAT A
CA AGT CCC CTA CTG GTA CTG CTA TTC GA
D D D D K

However, studies by the present inventors revealed that, when a fusion protein was expressed by using the cassette vector according to the above embodiment and then the enzymatic cleavage site was hydrolyzed with enterokinase, one leucine residue remained at the N-terminal of the target protein, and thus the desired target protein was not directly obtained. This problem has not been known to those skilled in the art so far. An additional step for the removal of a modified amino acid at the N-terminal is not desired from viewpoints of manufacturing costs of a target protein and efficiency.

### Description of the Invention

An object of the present invention is to provide a method for efficient expression of a target protein, and to provide a fusion protein that can be used for said method. More specifically, the object of the present invention is to provide a fusion protein that enables efficient production of a target protein by an enzymatic treatment, and a method for producing the target protein by using the aforementioned fusion protein. Another object of the present invention is to provide a DNA for the manufacture of the fusion protein having the aforementioned characteristics; a recombinant vector comprising the aforementioned DNA; and a transformant deriving from a mammal which comprises the aforementioned recombinant vector.

The inventors of the present invention conducted various researches on the fusion protein techniques in order to achieve the foregoing objects. As a result, they found that, when a fusion protein is expressed which is provided with a proteolytically cleavable peptide linker between the immunoglobulin Fc chain and a target protein, a target protein having no additional amino acid at its N-terminal can be produced very efficiently by the steps of expressing a fusion protein, in which the C-terminal of an enzymatic cleavage site present in the peptide linker is bound directly to the N-terminal of the target protein by means of a peptide bond, and then treating the fusion protein with a proteolytic enzyme. The present invention was achieved on the basis of these findings.

The present invention thus provides a fusion protein containing a target protein, which comprises, from its N-terminal to C-terminal direction:
a) a signal sequence;
b) an immunoglobulin Fc region which lacks at least the CH1 domain;
c) a peptide linker which contains an enzymatic cleavage site at its C-terminal; and
d) an amino acid sequence of the target protein,
characterized in that the target protein after an enzymatic cleavage does not have at its N-terminal one or more amino acid residues derived from the peptide linker. According to preferred embodiments of the present invention, there are provided the aforementioned fusion protein which is used for the production of the target protein; the aforementioned fusion protein wherein the target protein is granulocyte colony-stimulating factor; the aforementioned fusion protein wherein the target protein is erythropoietin; and the aforementioned fusion protein wherein the proteolytic enzyme is enterokinase or factor Xa.

According to another aspect of the present invention, there are provided a DNA which encodes the aforementioned fusion protein; a recombinant vector which comprises the aforementioned DNA; and a mammalian cell which is transformed with the aforementioned recombinant vector. According to further aspect of the present invention, there are provided a method for producing the aforementioned fusion protein, which comprises the step of collecting the aforementioned fusion protein from a culture of the aforementioned mammalian cell capable of producing the fusion protein; and a method for producing the target protein, which comprises the step of producing the target protein by an enzymatic treatment of the aforementioned fusion protein.

### Brief Explanation of the Drawing

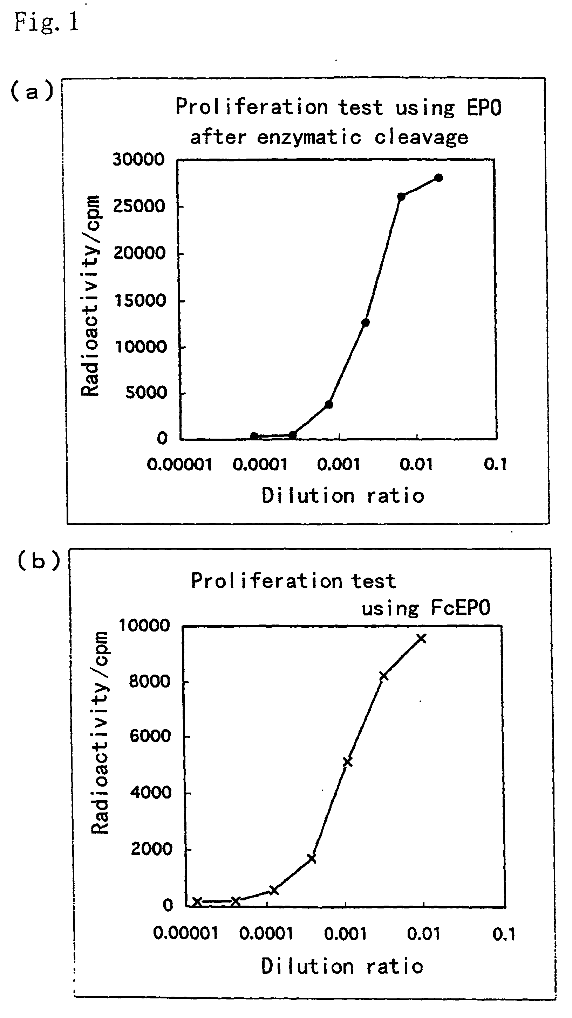

Figure 1 depicts cell proliferating activity of a fusion protein of the present invention comprising erythropoietin, and erythropoietin produced by an enzymatic treatment of the fusion protein. In the figure, (a) shows cell proliferating activity of erythropoietin after the enzymatic cleavage, and (b) shows cell proliferating activity of the fusion protein before the enzymatic cleavage.

### Best Mode for Carrying out the Invention

The fusion protein provided by the present invention is used for the production of a target protein, and it comprises, from its N-terminal to C-terminal direction, a) a signal sequence; b) an immunoglobulin Fc region which lacks at least the CH1 domain; c) a peptide linker containing a proteolytic cleavage site at its C-terminal; and d) an amino acid sequence of the target protein. The fusion protein of the present invention is based on a fusion protein disclosed in the specification of the U.S. Patent No. 5,541,087, and is characterized in that the target protein does not have one or more amino acid residues derived from the peptide linker at its N-terminal after the enzymatic cleavage. No disclosure is given in the specification of the aforementioned United States patent as to the necessity of preparation of a fusion protein having the above characteristics or a specific method for preparation thereof.

As the signal sequence and the immunoglobulin Fc region which lacks at least the CH1 domain, and as the target protein of the fusion protein provided by the present invention, those disclosed in the specification of the U.S. Patent No. 5,541,087, for example, may be used. Those skilled in the art should refer to the specification of the aforementioned United States patent to carry out the present invention, and its disclosure is herein incorporated by reference.

The signal sequence is not particularly limited, and any signal sequence may be used so long as it enhances or assists the transportation of an expressed fused protein across the cellular membrane and extracellular secretion. The number of amino acid residues is also not particularly limited, which is generally about 10 to 50, preferably about 15 to 30. The signal sequence may be either a naturally derived or a non-natural signal sequence. A signal sequence may also be referred to as a "signal peptide", "leader sequence", "leader peptide" or the like; however, its meaning is well known and definite to those skilled in the art (see, for example, Heijine, Nucleic Acids Res., 14, 4683, 1986).

For the fusion protein of the present invention, for example, antibody light (L) chain signal sequences, e.g., antibody 14.18 (Gillies, et al., J. Immunol. Method, 125, pp.191-202, 1989), antibody heavy (H) chain signal sequences, e.g., the MOPC141 antibody heavy chain signal sequence (Sakano et al., Nature, 286, 5774, 1980), and any other signal sequences which are well known in the art (see, for example, Watson, Nucleic Acids Research, 12, 5145, 1984) can be used. However, signal sequences which can be used for the production of the fusion protein of the present invention are not limited to the aforementioned specific examples. The signal sequence of the fusion protein of the present invention should be appropriately chosen so that the fusion protein can efficiently be secreted, and such secretion ability can be readily determined according to the method described in the examples given in the present specification.

The immunoglobulin Fc region is an amino acid sequence containing the carboxyl-terminal portion of an immunoglobulin H chain constant region. For example, the Fc region of IgA, IgD and IgG is a dimer of the hinge-CH2-CH3 domains, and the Fc region of IgM and IgE is a dimer of the hinge-CH2-CH3-CH4 domains. For the production of the fusion protein of the present invention, any Fc region can be used. In addition to the natural Fc regions, non-natural Fc regions produced by genetic recombination techniques or the like can be used. Such non-natural Fc regions may be so-called chimeric Fc regions comprising a combination of partial amino acid sequences derived from immunoglobulins of different classes, or those having substitution, insertion, and/or deletion of a part of amino acid residues that constitute the Fc region as explained below.

For example, it may sometimes be preferred to delete the binding sites for certain proteins from the Fc region. For example, since co-expression with the L chain is generally unnecessary, the binding site for the heavy chain binding protein, Bip (Hendershot et al., Immunol. Today, 8, pp.111-114, 1987), should desirably be deleted from the CH2 domain of the Fc region of IgE so as to achieve efficient secretion of the fusion protein. Likewise, the cysteine residues present in the Fc regions which are responsible for binding to the L chain of the immunoglobulin should be deleted or substituted with another amino acid, such that these cysteine residues do not interfere with the proper folding of the Fc region when the fusion protein is expressed. In the same manner, transmembrane domain sequences, such as those present in IgM, should preferably be deleted in a viewpoint of secretion efficiency.

The Fc region is known to have some of the biological properties, termed "effector functions". Typical examples of the effector functions include, for example, complement fixation, Fc receptor binding, binding to cell membranes, prolongation of blood half-life, and placental transfer. In the Fc region used for the production of the fusion protein of the present invention, one or more of these effector functions may be modified or removed. For example, one or more amino acids responsible for the effector functions identified by Duncan et al. (Nature, 332, 738, 1988) may be substituted or deleted (see also, for example, Yasmeen, et al., Immunol., 116, 518, 1976; Tao, et al., J. Immunol., 143, 2595, 143).

Preferred Fc regions are those derived from immunoglobulin γ, more preferably Fc regions derived from immunoglobulin γ-1. As the Fc regions of immunoglobulin γ-1, those containing at least a part of the hinge region, CH2 region, and CH3 region are preferred. Furthermore, Fc regions in which CH2 region is deleted are also preferred (Gillies, et al., Hum. Antibod. Hybridomas, 1, 47, 1990). However, the Fc regions which can be used for the production of the fusion protein of the present invention are not limited to those explained above, and an appropriate Fc region can be chosen by those skilled in the art. It can also be understood that modification or alteration of the Fc region may be readily achieved by those skilled in the art by applying molecular biology techniques well known in the art.

Types of the target protein are not particularly limited. The target protein can be chosen from any proteins; however, they may preferably be chosen from physiologically active proteins. As the physiologically active proteins, non-natural proteins which are produced by introducing substitution, insertion and/or deletion of one or several amino acid residues in amino acid sequences of natural proteins may be used in addition to natural proteins. The target protein may be a mature or a precursor protein.

Since one of the objects of the present invention is to conveniently produce a solubilized target protein in a large scale, use of a solubilized protein as the target protein is preferred according to the present invention. When a protein has a hydrophobic region, whose typical examples include transmembrane domains, the proteins can generally be solubilized by removing such hydrophobic regions. It is also possible to cleave only an extracellular domain to use as a solubilized protein. As the target protein for the production of the fusion protein of the present invention, cell growth factors such as erythropoietin, granulocyte colony-stimulating factor, and granulocyte macrophage colony-stimulating factor are preferred. Among them, erythropoietin and granulocyte colony-stimulating factor are more preferred. However, the target proteins for the production of the fusion protein of the present invention are not limited to these examples. The target protein may include a labeling agent such as biotin.

The number of amino acid residues that constitute the peptide linker containing a proteolytic cleavage site at its C-terminal is not particularly limited. It is preferred to use a linker comprising, for example, about 5 to 50 amino acid residues. The peptide linker may have one or more other enzymatic cleavage sites in addition to the enzymatic cleavage site at the C-terminal. The types of enzymes used for the enzymatic cleavage are not particularly limited so far that the enzymes can specifically recognize the enzymatic cleavage site present in the peptide linker and cleave it under mild conditions. Generally, the enzymes can be chosen from endopeptidases. Examples include trypsin, chymotrypsin, elastase, pepsin, papain, subtilisin, thermolysin, enterokinase, and factor Xa.

In the fusion protein of the present invention, the enzymatic cleavage site located at the C-terminal portion of the peptide linker must be chosen so that one or more amino acid residues derived from the peptide linker do not remain at the N-terminal of the target protein after the enzymatic cleavage. Generally, it is preferred to choose an endopeptidase that specifically recognizes an enzymatic cleavage site consisting of one or more amino acid residues and cleaves the enzymatic cleavage site at its C-terminal. In that case, for the preparation of a DNA encoding the fusion protein of the present invention, when a DNA sequence encoding a part or full length of the peptide linker is ligated to a DNA sequence encoding a part or entire target protein, the peptide sequence encoded by the ligated DNA sequence should be designed so that the C-terminal of the enzymatic cleavage site consisting of one or more amino acid residues can be directly bound to the N-terminal of the target protein. For example, when a DNA encoding an oligopeptide lacking a C-terminal portion of the peptide linker is used, it is necessary to add one or more nucleotides to the 5' end of the DNA encoding the target protein so that the ligated DNA sequence can encode a complete peptide linker.

For example, a linker containing an enzymatic cleavage site that is cleavable by enterokinase (enterokinase linker), and a linker containing an enzymatic cleavage site that is cleavable with factor Xa (factor Xa linker) will be specifically explained. In the enterokinase linker, a sequence of Asp-Asp-Asp-Asp-Lys is provided at the C-terminal as the enzymatic cleavage site, and the N-terminal of the target protein can be directly bound to the lysine residue. In the factor Xa linker, a sequence of Ile-Glu-Glu-Arg is provided at the C-terminal of the linker as the enzymatic cleavage site, and the N-terminal of the target protein can be directly bound to the arginine residue. When the fusion proteins of the above structures are treated each with enterokinase or factor Xa, each of the C-terminal sites of the oligopeptides consisting of 4 or 5 amino acid residues as the enzymatic cleavage sites is hydrolyzed, and a target protein can be obtained which has no remaining amino acid residue at the N-terminal that is derived from the peptide linker.

The DNAs of the present invention provided by another aspect are specified as nucleic acid sequences encoding amino acid sequences of the fusion proteins mentioned above, and the sequences are not particularly limited so long as they encodes amino acid sequences of the aforementioned fusion proteins. Vectors used for the preparation of the recombinant vector provided by the present invention may be any types of vectors so long as they can transfer the aforementioned DNA into mammalian cells so that the fusion protein of the present invention can be expressed from the DNA. Various vectors are known which can be used for the above purpose, and those skilled in the art can choose a suitable vector depending on types of an animal cell used, a target protein, an enzymatic cleavage site and the like. The vector may contain one or more marker genes. Types of animal cells to be subjected to the transfer of the recombinant vector are also not particularly limited.

It is readily understood that techniques used for the preparations of the fusion protein, the DNA, the recombinant vector, and the transformant deriving from mammalian cell of the present invention are well known and widely used in the art. Methods for preparation of the fusion protein of the present invention and expression thereof, and methods for preparation of a target protein will be specifically explained and detailed in Examples of the specification. Those skilled in the art will understand that any desired fusion protein can be readily produced by referring to the foregoing general explanation and specific description in Examples, and applying appropriate modifications or alterations as required.

### Examples

The present invention will be explained more specifically with reference to the following examples. However, the scope of the present invention is not limited to the examples.

### Example 1: Cloning of EPO cDNA

In this example, a secretion cassette vector comprising erythropoietin (referred to as "EPO" hereinafter in the examples) gene was prepared based on the secretion cassette described in Example 1 of the specification of the U.S. Patent No. 5,541,807, and a fusion protein comprising the target protein and the immunoglobulin Fc chain was expressed. The secretion cassette described in the specification of the U.S. Patent No. 5,541,807 has a DNA sequence encoding the immunoglobulin L chain signal peptide and the immunoglobulin Fc chain from its 5' to 3' direction, and the cassette can be inserted with a DNA having the Xma restriction site at its 3' side.

The following two PCR primers (PR1, PR2) were synthesized based on a known DNA sequence of the mature EPO (GeneBank X02157), and cDNA of EPO was amplified by utilizing the well known PCR technique.
PR1: 5'-GGCTCCTCCACGGCTCATCTGTGAC
PR2: 5'-GCTATAACCTCGAGTCATCTGTCCCCTGTC (Biotin was bound at the 5' end)

The PCR amplification product was captured with avidin beads (Dynabeads), and collected after digestion with restriction enzyme Ava I.

DNAs (L1, L2) encoding a portion of enterokinase enzymatic cleavage site (DDDDK) except its C-terminal and containing the XmaI restriction site in the upstream were synthesized.
L1: CCGGGCTCTGGCGATGACGATGACAA
L2: TTGTCATCGTCATCGCCAGAGC

L1, L2 and the aforementioned PCR product were inserted into the XmaI/XhoI site of the secretion cassette to prepare a recombinant vector (CMVFcEPO#EK) that was capable of expressing the fusion protein of the present invention. This fusion protein contained, from its N-terminal, the immunoglobulin L chain, signal peptide and the immunoglobulin Fc chain, the peptide linker provided with the enterokinase enzymatic cleavage site at its C-terminal, and EPO whose N-terminal was bound to the C-terminal of the enzymatic cleavage site.

In a similar manner, a recombinant vector (CMVFcEPO#Xa) that was capable of expressing another fusion protein of the present invention was produced by utilizing DNAs encoding a portion of the factor Xa enzymatic cleavage site (IEGR) except its C-terminal, and containing the XmaI restriction site in the upstream (L3, L4). This fusion protein contained, from its N-terminal, the immunoglobulin L chain signal peptide and the immunoglobulin Fc chain, the peptide linker provided with the factor Xa enzymatic cleavage site at its C-terminal, and EPO whose N-terminal was bound to the C-terminal of the enzymatic cleavage site.
L3: CCGGGCTCTGGCATCGAGGGGAG
L4: CTCCCCTCGATGCCAGAGC

In the table shown below, structures of the enzymatic cleavage sites in the peptide linkers and the N-terminal portions of the EPO bound to the C-terminals of the linkers in the aforementioned fusion proteins, and nucleic acid sequences contained in the aforementioned recombinant vectors are shown, wherein the underlined portions indicate the enzymatic cleavage sites, and the amino acid sequences of Ala-Pro-Pro-Arg--- after the cleavage sites represent the N-terminal portion of EPO. In the nucleic acid sequences, the capital letters indicate portions derived from L1 and L2, or L3 and L4, and small letters indicate portions derived from the PCR amplification products.

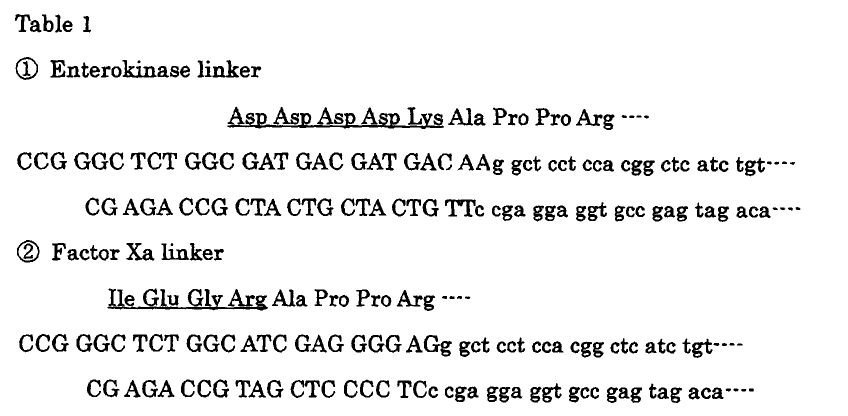

### Example 2: Expression of fusion protein

Transfer of the CMVFcEPO prepared in Example 1 into animal cells was performed by the protoplast fusion technique (described in Molecular Cloning, 2nd Ed., Chapter 16, Ed. by Cold Spring Harbor Laboratory). Escherichia coli (C600) cells transformed with CMVFcEPO plasmid were cultured, and treated with lysozyme to obtain protoplasts, which were then fused with NS0 cells. The fused cells were cultured in a selection medium (DMEM culture medium containing 0.1 µM methotrexate, and 10% fetal bovine serum) from two days after the fusion, and clones expressing the fusion protein of the present invention which contained EPO (FcEPO) were screened by subjecting the culture supernatants of the wells in which colonies appeared to Fc sandwich ELISA measurement (Antihuman IgG/Antihuman Fc-POD, Jackson).

FcEPO was identified by the immunoprecipitation described in Antibodies, A Laboratory Manual (Cold Spring Harbor Laboratory Ed.) and Western blotting. Protein A beads (Pharmacia) were added to each of the culture supernatants, and after stirring overnight, adsorbed proteins were eluted with Laemmli's buffer and then subjected to SDS-polyacrylamide gel electrophoresis (SDS-PAGE). As a result, a band of molecular weight of 64 kD that corresponded to FcEPO was observed. The band was blotted to a PVDF membrane (Millipore), and reacted with peroxidase labeled Antihuman IgG (Jackson). Similarly, the band was also reacted with Antihuman EPO mouse monoclonal antibodies (Jackson) × peroxidase labeled antimouse IgG. For each system, the peroxidase activity was measured by using LAS1000 from Fuji Photo Film Co., Ltd. based on a chemiluminescence method (ECL, Amesrham). As a result, the expression of FcEPO was observed.

In the same manner, the transfer and expression system of CMVFcEPO gene in CHO cells was constructed. Cells exhibiting stable expression established as described above (1,000,000 cells/ml) were cultured for 7 days, and amounts of FcEPO secreted in culture supernatants were assayed by ELISA. The results are shown in Table 2 below. These results indicate that the expression according to the present invention is a protein expression system achieving extremely high expression levels.

**Table 2**

| Clone | Parent cell | Production yield (µg/ml) | Enzymatic cleavage site |
|---|---|---|---|
| E10 | NS0 | 60 | DDDDK |
| B5 | NS0 | 100 | IEGR |
| CH | CHO | 50 | DDDDK |

### Example 3: Cleavage of enzymatic cleavage site by enterokinase

The FcEPO expression clone E10 established in Example 2 was mass cultured in a spinner flask, and the product was purified with protein A. The purified FcEPO (0.7 µg/ml, 50 mM Tris, 1 mM CaCl₂, 0.1% Tween, pH 8 buffer) was added with enterokinase (Invitrogen) to a final concentration of 3.3 units/ml, and the mixture was allowed to react overnight at room temperature and then subjected to SDS-PAGE. As a result, the 64 kD band corresponding to the FcEPO disappeared, and bands corresponding to Fc and EPO (about 32 kD) were observed. The results clearly demonstrate that the peptide linker in the FcEPO was cleaved by the enterokinase.

### Example 4: Assay of cell proliferation enhancing activity

Cell proliferation enhancing activity of the FcEPO and the enzymatically hydrolized product was measured by the method for measuring EPO activity described in Current Protocols in Immunology 6.17.1. TF1 cells in culture were washed with a culture medium, and added to wells of a 96-well plate. The EPO after the enzymatic cleavage or the FcEPO before the enzymatic cleavage obtained in Example 3 were added to the wells and then cultivation was continued for 2 nights. The cells were further added with ³H-thymidine and cultured for 4 hours, and then incorporated ³H-thymidine was measured by a liquid scintillation counter. The results are shown in Figure 1. These results clearly show that the EPO after the enzymatic cleavage was highly active in cell proliferation enhancing activity.

### Example 5: Screening system for EPO/EPO receptor binding inhibitor

TF1 cells, FcEPO, or EPO (already cleaved with enterokinase), and a binding inhibitor (an antibody) were mixed in ratios shown in Table 3 below, and cultured in a carbon dioxide incubator for 2 days.

**Table 3**

| | 1 | 2 | 3 | 4 |
|---|---|---|---|---|
| FcEPO | 50 µl | | 50 µl | |
| EPO | | 50 µl | | 50 µl |
| Inhibitor | Culture medium 50 µl | Culture medium 50 µl | Anti-EPO antibody 50 µl | Anti-EPO antibody 50 µl |
| Cell (TF1) | 100 µl | 100 µl | 100 µl | 100 µl |

The cells were further added with ³H-thymidine and cultivation was continued for 4 hours, and then incorporated ³H-thymidine was measured by a liquid scintillation counter. As a result, counts were 25,653 for Experiment 1, 24,358 for Experiment 2, 1,250 for Experiment 3, and 1,800 for Experiment 4. It was found that the anti-EPO antibody inhibited the binding of EPO to the EPO receptor, and consequently inhibited proliferation of TF1. These results indicate that FcEPO according to the present invention and EPO cleaved from FcEPO can be used in a screening system for a receptor/ligand binding inhibitor.

### Example 6: Construction of GCSF expression system

Construction of granulocyte colony stimulating factor (GCSF) expression system was performed in a manner similar to that of the EPO cloning in Example 1. The following two PCR primers (PR3, PR4) were synthesized based on a known DNA sequence of the mature GCSF, and cDNA of GCSF was amplified by utilizing the well known PCR technique.
PR3: ACCCCCCTCGGACCTGCCAGCTC
PR4: CGTGCAGAATTCGGCTTCTCGAGTCAA (Biotin was bound at the 5' end)

The PCR amplified product was captured with avidin beads (Dynabeads), and collected after digestion with restriction enzyme Ava I.

DNAs encoding a portion of enterokinase enzymatic cleavage site (DDDDK) and containing the XmaI restriction site in the 5' side (L5, L6) were synthesized.
L5: CCGGGCTCTGGCGATGACGATGACAAG
L6: CTTGTCATCGTCATCGCCAGAGC

L5, L6 and the aforementioned PCR product were inserted into the XmaI/XhoI site of the secretion cassette to prepare a recombinant vector (CMVFcGCSF#EK) that was capable of expressing the fusion protein of the present invention. The fusion protein contained, from its N-terminal, the immunoglobulin L chain signal peptide and the immunoglobulin Fc chain, the peptide linker provided with the enterokinase enzymatic cleavage site at its C-terminal, and GCSF whose N-terminal was bound to the C-terminal of the enzymatic cleavage site.

In a similar manner, a recombinant vector (CMVFcGCSF#Xa) which was capable of expressing a fusion protein of the present invention was produced by utilizing DNAs encoding the factor Xa enzymatic cleavage site (IEGR), and containing the XmaI restriction site at the 5' side (L7, L8). This fusion protein contained, from its N-terminal, an immunoglobulin L chain signal peptide and the immunoglobulin Fc chain, the peptide linker provided with the factor Xa enzymatic cleavage site at its C-terminal, and GCSF whose N-terminal was bound to the C-terminal of the enzymatic cleavage site.
L7: CCGGGCAGCGGCATCGAGGGCAGA
L8: TCTGCCCTCGATGCCGCTGC

### Example 7: Expression of GCSF fusion protein

The vector for expression of the GCSF fusion protein produced in Example 6 was transferred to 293 cells by the calcium phosphate method, and the expression of the fusion protein was verified by Western blotting after one week.

### Industrial Applicability

The fusion protein of the present invention is characterized in that it enables extremely efficient expression in a large scale and the manufacture of a target protein having no amino acid-modification at its N-terminal in a high yield and only with a simple enzymatic treatment.

## Claims

1. A fusion protein containing a target protein, which comprises, from its N-terminal to C-terminal direction:
a) a signal sequence;
b) an immunoglobulin Fc region which lacks at least the CH1 domain;
c) a peptide linker which contains an enzymatic cleavage site at its C-terminal; and
d) an amino acid sequence of the target protein,
characterized in that the target protein after an enzymatic cleavage does not have at its N-terminal one or more amino acid residues derived from the peptide linker.

2. The fusion protein of claim 1, which is used for the production of the target protein.

3. The fusion protein of claim 1 or 2, wherein the target protein is granulocyte colony stimulating factor.

4. The fusion protein of claim 1 or 2, wherein the target protein is erythropoietin.

5. The fusion protein of any one of claims 1 to 4, wherein an enzyme that cleaves the enzymatic cleavage site is enterokinase or factor Xa.

6. A DNA which encodes a fusion protein of any one of claims 1 to 5.

7. A recombinant vector which comprises the DNA of claim 6.

8. A mammalian cell for producing a fusion protein of any one of claims 1 to 5, which is transformed with the recombinant vector of claim 7.

9. A method for producing a fusion protein of any one of claim 1 to 5, which comprises the step of collecting the fusion protein from a culture of the mammalian cell of claim 8.

10. A method for producing a target protein, which comprises the step of treating a fusion protein of any one of claim 1 to 5 with an enzyme.

11. The method of claim 10, which comprises the step of treating a fusion protein produced by the method of claim 9 with an enzyme.


SEQUENCE LISTING

<110> Fuji Photo Film Co., Ltd.

<120> Fusion Protein

<130> 99164M

<150> JP P1998-132389

<160> 12

<210> 1

<211> 25

<212> DNA

<213> Artificial Sequence

<400> 1

ggctcctcca cggctcatct gtgac 25

<210> 2

<211> 30

<212> DNA

<213> Artificial Sequence

<400> 2

gctataacct cgagtcatct gtcccctgtc 30

<210> 3

<211> 26

<212> DNA

<213> Artificial Sequence

<400> 3

ccgggctctg gcgatgacga tgacaa 26

<210> 4

<211> 22

<212> DNA
<213> Artificial Sequence
<400> 4
ttgtcatcgt catcgccaga gc 22
<210> 5
<211> 23
<212> DNA
<213> Artificial Sequence
<400> 5
ccgggctctg gcatcgaggg gag 23
<210> 6
<211> 19
<212> DNA
<213> Artificial Sequence
<400> 6
ctcccctcga tgccagagc 19
<210> 7
<211> 23
<212> DNA
<213> Artificial Sequence
<400> 7
acccccctcg gacctgccag ctc 23
<210> 8
<211> 27
<212> DNA
<213> Artificial Sequence
<400> 8

cgtgcagaat tcggcttctc gagtcaa 27
<210> 9
<211> 27
<212> DNA
<213> Artificial Sequence
<400> 9
ccgggctctg gcgatgacga tgacaag 27
<210> 10
<211> 23
<212> DNA
<213> Artificial Sequence
<400> 10
cttgtcatcg tcatcgccag agc 23
<210> 11
<211> 24
<212> DNA
<213> Artificial Sequence
<400> 11
ccgggcagcg gcatcgaggg caga 24
<210> 12
<211> 20
<212> DNA
<213> Artificial Sequence
<400> 12
tctgccctcg atgccgctgc 20